# EUROPÄISCHE PATENTSCHRIFT

(11) **EP 2 211 751 B1**
(45) Veröffentlichungstag und Bekanntmachung des Hinweises auf die Patenterteilung: **20.09.2017**
(21) Anmeldenummer: 08852259.4
(22) Anmeldetag: 18.11.2008
(51) Int. Cl.: A61B 34/20, A61B 34/30, A61B 90/00

(54) **EINEN ROBOTER AUFWEISENDE VORRICHTUNG, MEDIZINISCHER ARBEITSPLATZ UND VERFAHREN ZUM REGISTRIEREN EINES OBJEKTS**
DEVICE COMPRISING A ROBOT, MEDICAL WORK STATION, AND METHOD FOR REGISTERING AN OBJECT
DISPOSITIF PRÉSENTANT UN ROBOT, POSTE DE TRAVAIL MÉDICAL ET PROCÉDÉ POUR ENREGISTRER UN OBJET

(30) Priorität: 19.11.2007 DE 102007055203
(43) Veröffentlichungstag der Anmeldung: 04.08.2010
(73) Patentinhaber: KUKA Roboter GmbH, 86165 Augsburg (DE)
(72) Erfinder: ORTMAIER, Tobias, 30966 Hemmingen (DE); JACOB, Dirk, 87616 Marktoberdorf (DE); NEFF, Thomas, 81663 Augsburg (DE)
(74) Vertreter: Ege Lee & Partner Patentanwälte PartGmbB
(86) Internationale Anmeldenummer: PCT/EP2008/065753
(87) Internationale Veröffentlichungsnummer: WO 2009/065827

(56) Entgegenhaltungen:
- EP-A1- 1 754 448
- WO-A-2007/003949
- WO-A-2008/058520
- WO-A1-99/50721
- DE-A1- 10 249 786
- US-A- 5 868 675
- US-A1- 2004 111 183
- US-A1- 2007 013 336
- US-A1- 2007 078 473
- US-A1- 2007 129 846

## Beschreibung

Die Erfindung betrifft eine, einen Roboter aufweisende Vorrichtung, einen medizinischen Arbeitsplatz und ein Verfahren zum Registrieren eines Objekts.

Die US 2004/0077939 A1 offenbart einen medizinischen Arbeitsplatz mit einem Röntgengerät, einem chirurgischen Instrument, einem Positionserfassungssystem und einem das chirurgische Instrument führenden Roboter zur Behandlung eines Patienten in zumindest teilweise automatisierter Weise. Um die Positionen des chirurgischen Instruments, des Röntgengerätes und des Patienten zu erfassen, sind am Röntgengerät, am Patienten und am chirurgischen Instrument oder am Roboter Positionsmarker angeordnet, die von einer optischen Positionserfassungsvorrichtung des Positionserfassungssystems aufgenommen werden. Aufgrund einer Auswertung der mit der optischen Positionserfassungsvorrichtung aufgenommenen Bilder der Positionsmarker kann die Position und gegebenenfalls auch die Orientierung der Positionsmarker und somit des chirurgischen Instruments, des Röntgengerätes und des Patienten im Raum ermittelt werden.

Die US 6,228,089 B1 offenbart eine Vorrichtung zum Positionieren und Führen eines chirurgischen Werkzeugs bei orthopädischen Eingriffen, die einen Industrieroboter mit einem programmgesteuerten, mehrgelenkigen und eine Montageplatte umfassenden Roboterarm und einer manuell führbaren, an der Montageplatte befestigten Sensoreinrichtung für eine dreidimensionale Objektvermessung im Werkzeug-Koordinatensystem des Industrieroboters aufweist. Die Sensoreinrichtung umfasst einen manuell führbaren, mehrgelenkigen Tastarm mit Sensoren zur Erfassung der Gelenkstellungen oder eine Messanordnung mit Datenfernübertragung, die einen stationären Empfänger, einen Taststift mit einem Sender und einen an der Montageplatte befestigten Sender aufweist. Die Sender umfassen mehrere Signalgeber in einer fest vorgegebenen räumlichen Zuordnung, die von dem Empfänger aufgenommene Ortungssignale abgeben.

Die WO 2007/003949 A1 offenbart einen medizinischen Arbeitsplatz, der eine Patientenliege, ein Navigationssystem und einen Roboterarm aufweist. Am Roboterarm ist eine Kamera des Navigationssystems befestigt.

Die Aufgabe der Erfindung ist es, Voraussetzungen für einen medizinischen Arbeitsplatz mit einem Roboter zu schaffen, so dass Positionen des Roboters und wenigstens eines weiteren Objekts genauer bestimmt werden können.

Eine weitere Aufgabe der Erfindung ist es, eine, einen Roboter aufweisende Vorrichtung für einen entsprechend eingerichteten medizinischen Arbeitsplatz anzugeben.

Die Aufgabe der Erfindung wird gelöst durch eine Vorrichtung gemäß dem Patentanspruch 1.

Handelt es sich bei dem Objekt um ein Lebewesen, dann wird die Aufgabe der Erfindung auch gelöst durch einen medizintechnischen Arbeitsplatz, aufweisend die erfindungsgemäße Vorrichtung und eine Patientenliege, die vorgesehen ist, dass auf ihr das Lebewesen als das Objekt liegt.

Navigationssysteme sind in der Medizintechnik, insbesondere in der minimalinvasiven Medizintechnik, beispielsweise aus der US 6,895,268 B1 allgemein bekannt. Navigationssysteme umfassen die Erfassungsvorrichtung, die beispielsweise eine optische Erfassungsvorrichtung, insbesondere eine Kamera, ein Lasertrackingsystem, Projektoren für ein strukturiertes Licht oder Linienprojektoren aufweisen können. Die Erfassungsvorrichtung ist eingerichtet, in allgemein bekannter Weise, die am Objekt, insbesondere an der Oberfläche des Objekts, angeordneten Marker oder markanten Stellen der Oberfläche des Objekts zu erfassen. Aufgrund der mit der Vorrichtung zum Erfassen der Marker bzw. der markanten Stellen kann eine Rechenvorrichtung des Navigationssystems in im Wesentlichen allgemein bekannter Weise die Position des Lebewesens und gegebenenfalls dessen Orientierung bestimmen. Erfindungsgemäß ist die Erfassungsvorrichtung des Navigationssystems am Roboter befestigt. Aufgrund der Befestigung der Erfassungsvorrichtung am Roboter ist die Stellung der Koordinatensysteme des Navigationssystems und des Roboters zueinander bekannt, wodurch das Navigationssystem die Position und gegebenenfalls die Orientierung des Roboters nicht mittels Marker beziehungsweise markanter Stellen des Roboters zu bestimmen braucht. Dadurch ergeben sich Voraussetzungen, dass die Position des Objekts insbesondere bezüglich des Roboters genauer bestimmt werden kann.

Erfindungsgemäß ist die Erfassungsvorrichtung integraler Bestandteil eines Roboterarms des Roboters oder ist an diesem starr, aber lösbar befestigt.

Roboter im Allgemeinen sind Handhabungsmaschinen, die zur selbsttätigen Handhabung von Objekten mit zweckdienlichen Werkzeugen ausgerüstet und bezüglich mehrerer Bewegungsachsen, insbesondere hinsichtlich Orientierung, Position und Arbeitsablauf programmierbar sind. Roboter umfassen im Allgemeinen den Roboterarm, der auch als Manipulator bezeichnet wird, eine Steuervorrichtung und gegebenenfalls einen Effektor, der zum Beispiel als ein Greifer zum Greifen eines Werkzeugs oder, wenn zum Beispiel in der Medizintechnik angewandt, zum Greifen eines medizinischen Instruments, insbesondere eines chirurgischen Instruments, ausgebildet sein kann. Das medizinische Instrument kann auch über einen Adapter am Effektor befestigt sein. Der Manipulator beziehungsweise der Roboterarm stellt im Wesentlichen den beweglichen Teil des Roboters dar. Der Roboterarm weist insbesondere mehrere Achsen auf, die zum Beispiel mittels elektrischer Antriebe von der zum Beispiel als Rechner ausgeführten Steuervorrichtung angesteuert werden.

Nach einer Ausführungsform der erfindungsgemäßen medizintechnischen Vorrichtung weist deren Roboter eine am Roboter befestigte Beleuchtung auf. Die Beleuchtung ist beispielsweise am Roboterarm des Roboters befestigt oder in diesen integriert.

Aufgrund der am Roboter, insbesondere an dessen Roboterarm befestigten Erfassungsvorrichtung des Navigationssystems sind beispielsweise Voraussetzungen für eine verbesserte Zugänglichkeit des Erfassungssystems insbesondere im Operationsbereich gegeben, da die Lage des Erfassungssystems durch eine Bewegung des Roboters geändert und somit an Bedingungen im Operationsbereich angepasst werden kann. Wird die erfindungsgemäße Vorrichtung beziehungsweise der erfindungsgemäße medizinische Arbeitsplatz für eine navigierende Operation verwendet wird, dann kann beispielsweise der Roboter automatisch einen relativ günstigen, insbesondere optimalen Blickwinkel für die Erfassungsvorrichtung wählen, damit diese relativ gut die Marker beziehungsweise die markanten Stellen aufnehmen kann. Auch die optionale Beleuchtung kann entsprechend mitgeführt werden.

Da der Roboter über redundante Freiheitsgrade verfügt, ist auch eine Änderung des Blickwinkels der Erfassungsvorrichtung bei gleichzeitiger Beibehaltung des sogenannten Tool Center Points (TCP) möglich. Dies ist insbesondere dann vorteilhaft, wenn am Roboter das medizinische, insbesondere das chirurgische Instrument befestigt ist. Das medizinische, insbesondere chirurgische Instrument kann beispielsweise für eine automatische Operation des als Lebewesen ausgeführten Objekts mittels des Roboters verwendet werden.

Der Roboter ist derart eingerichtet, die Marker beziehungsweise die markanten Stellen des Objekts automatisch zu registrieren. Somit ergibt sich gemäß eines weiteren Aspekts der vorliegenden Erfindung ein Verfahren zum Registrieren eines Objekts, das folgenden Verfahrensschritt aufweist: automatisches Registrieren von markanten Stellen eines Objekts oder von an der Oberfläche des Objekts angeordneten Markern mittels eines Roboters, an dem eine Erfassungsvorrichtung eines Navigationssystems zum Erfassen der markanten Stellen beziehungsweise der am Objekt angeordneten Marker befestigt ist, wobei das Navigationssystem eine Verarbeitungsvorrichtung zum Ermitteln der Position des Objekts aufgrund der mit der Erfassungsvorrichtung erfassten markanten Stellen beziehungsweise Marker aufweist.

Um die Position des Objektes zu bestimmen, bestimmt das Navigationssystem die Positionen der Marker beziehungsweise der markanten Stellen. Um dies bewerkstelligen zu können, kann es nötig sein, die Marker beziehungsweise markanten Stellen zu registrieren. Dies wird gemäß einer Ausführungsform der erfindungsgemäßen medizintechnischen Vorrichtung dadurch durchgeführt, dass dessen Roboter eingerichtet ist, die Marker beziehungsweise die markanten Stellen durch Aufsuchen zu registrieren. Gemäß einer weiteren Ausführungsform der erfindungsgemäßen Vorrichtung ist dessen Roboter eingerichtet, die Marker beziehungsweise die markanten Stellen durch Aufnehmen zu registrieren. Die Aufnahme der Marker beziehungsweise der markanten Stellen kann insbesondere durch die Erfassungsvorrichtung des Navigationssystems erfolgen.

Wenn nach einer weiteren Ausführungsform der erfindungsgemäßen medizintechnischen Vorrichtung deren Roboter eingerichtet ist, eine Bewegung des Objekts zu erkennen und aufgrund der erkannten Bewegung automatisch die Positionen der Marker beziehungsweise der markanten Stellen zu aktualisieren, dann ergeben sich Voraussetzungen für eine verbesserte Bestimmung der Position des Objekts.

Der Roboter ist eingerichtet, nach bestimmten Zeitabständen, beispielsweise periodisch, automatisch die Marker beziehungsweise die markanten Stellen neu zu registrieren, gegebenenfalls deren Positionen zu bestimmen.

Mittels der erfindungsgemäßen Vorrichtung ist somit gegebenenfalls eine automatische Registrierung des Objekts, insbesondere des Lebewesens als das Objekt mit Hilfe der am Roboter befestigten Erfassungsvorrichtung möglich. Es können beispielsweise auch optional Algorithmen zur Gütebewertung der mittels der Erfassungsvorrichtung erfassten Daten zum Einsatz kommen. Somit kann der Aufwand und gegebenenfalls die Fehlerquote einer manuellen Registrierung verringert werden. Auch ist gegebenenfalls bei einer Lageänderung durch eine Bewegung des Objekts beziehungsweise des Lebewesens ein relativ einfaches Nachregistrieren möglich, das beispielsweise einmalig auf Basis eines bestimmten Vorgangs durchgeführt werden kann oder das periodisch stattfinden kann und damit automatisch mögliche Positionsveränderungen korrigieren kann.

Aufgrund der Befestigung der Erfassungsvorrichtung an den Roboter, der auch von Hand geführt werden kann, sind verschiedene Modi der Registrierung möglich. So ist beispielsweise ein manuelles Registrieren über das manuelle Anfahren von definierten Punkten, das heißt von markanten Stellen oder am Objekt angeordneten Marker am Eingriffsort, ein halbautomatisches Registrieren, bei dem definierte Punkte manuell angefahren werden und Zwischenstrecken automatisch erfasst beziehungsweise gescannt werden sowie eine vollautomatische Registrierung möglich, bei der der gesamte Registriervorgang ohne manuelles Eingreifen durchgeführt wird.

Die erfindungsgemäße Vorrichtung kann auch mit mehreren Robotern beziehungsweise mehreren Roboterarmen zusammenarbeiten, wobei insbesondere die Positionen der weiteren Roboterarme bekannt sind.

Die mittels der Erfassungsvorrichtung gewonnenen Bilddaten können beispielsweise auch mit präoperativ gewonnenen Bilddatensätze, die insbesondere mittels eines bildgebenden medizintechnischen Gerätes vom Objekt aufgenommen wurden, kombiniert werden. Geeignete bildgebende medizintechnische Geräte, die insbesondere dreidimensionale Bilddatensätze vom Objekt erstellen können, sind u. A. Magnetresonanzgeräte, Computertomographiegeräte, Röntgengeräte oder Ultraschallgeräte. Ebenso können alternativ oder zusätzlich Daten aus einem CAD System verwendet werden. Dadurch ist eine Anreicherung dieser Bilddatensätze durch die zusätzlich gewonnenen, mittels der Erfassungsvorrichtung aufgenommenen Daten möglich, womit beispielsweise einem Chirurgen die Arbeit entsprechend erleichtert werden kann.

Ein Ausführungsbeispiel der Erfindung ist exemplarisch in den beigefügten schematischen Zeichnungen dargestellt. Es zeigen:
- Fig. 1: einen Roboter,
- Fig. 2: eine Erfassungsvorrichtung eines Navigationssystems,
- Fig. 3: einen medizinischen Arbeitsplatz
- Fig. 4: ein bildgebendes medizintechnisches Gerät und
- Fig. 5: ein Flussdiagramm zur Veranschaulichung eines Registriervorgangs.

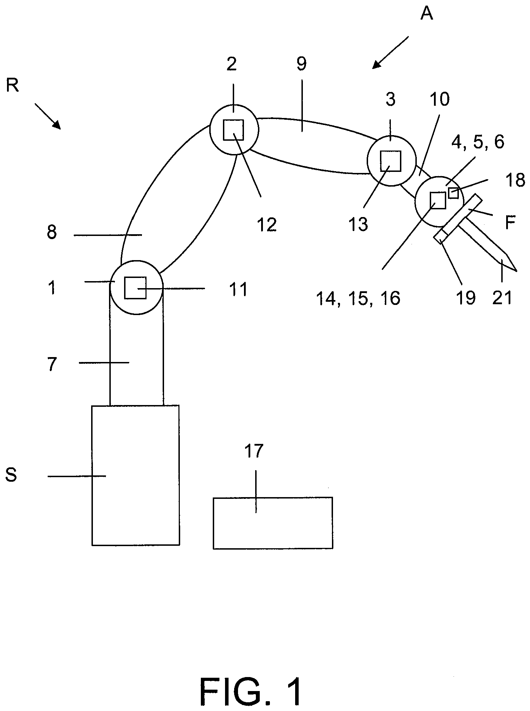

Die Fig. 1 zeigt einen Roboter R mit einem Roboterarm A, der im Falle des vorliegenden Ausführungsbeispieles auf einem Sockel S befestigt ist. Der Roboterarm A stellt im Wesentlichen den beweglichen Teil des Roboters R dar und umfasst mehrere Achsen 1 - 6, mehrere Hebel 7 - 10 und einen Flansch F, an dem im Falle des vorliegenden Ausführungsbeispieles ein chirurgisches Instrument 21 über einen nicht näher dargestellten Adapter befestigt ist. Das chirurgische Instrument 21 ist beispielsweise ein Endoskop.

Jede der Achsen 1 - 6 wird im Falle des vorliegenden Ausführungsbeispieles mit einem elektrischen Antrieb 11-16 bewegt, die in nicht dargestellter Weise mit einem Steuerrechner 17 des Roboters R elektrisch verbunden sind, so dass der Steuerrechner 17 bzw. ein auf dem Steuerrechner 17 laufendes Rechnerprogramm die elektrischen Antriebe derart ansteuern kann, dass die Position und Orientierung des Flansches F des Roboters R im Wesentlichen frei im Raum ausgerichtet werden kann. Die elektrischen Antriebe 11-16 des Roboters R umfassen z.B. jeweils einen elektrischen Motor und gegebenenfalls eine die Motoren ansteuernde Leistungselektronik.

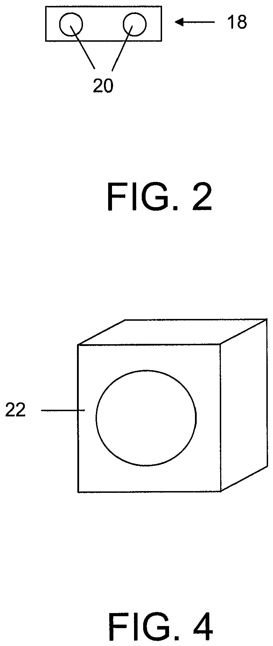

Im Falle des vorliegenden Ausführungsbeispieles umfasst der Roboter R ferner eine in der Fig. 2 näher dargestellte Erfassungsvorrichtung 18 eines Navigationssystems. Navigationssysteme als solche sind dem Fachmann u.A. aus der US 6,895,268 B1 bekannt und sind dafür vorgesehen, die Position eines Objekts, beispielsweise eines Lebewesens oder eines medizinischen Instrumentes, zu bestimmen.

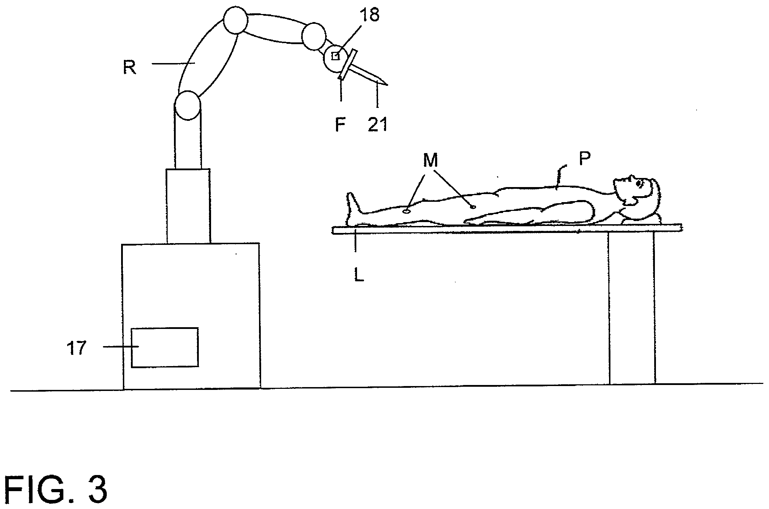

Navigationssysteme können beispielsweise magnetische oder optische Navigationssysteme sein und werden beispielsweise dafür eingesetzt, die Position und gegebenenfalls die Orientierung eines Objekts, beispielsweise eines in der Fig. 3 auf einer Patientenliege L liegenden Patienten P, zu ermitteln.

Um die Position des Patienten P zu ermitteln, sind an diesem bzw. an dessen Oberfläche z.B. Marker M angebracht, deren Positionen das Navigationssystem ermittelt. Die Lage der Marker, also deren Positionen und Orientierungen, relativ zum Patienten P muss dafür bekannt sein und wird in allgemein bekannter Weise ermittelt.

Im Falle des vorliegenden Ausführungsbeispieles weist die Erfassungsvorrichtung 18 eine Stereokamera 20 auf, die Bilder von den Markern M aufnimmt. Die Erfassungsvorrichtung 18 bzw. deren Stereokamera 20, ist im Falle des vorliegenden Ausführungsbeispieles in nicht dargestellter Weise mit dem Steuerrechner 17 des Roboters R verbunden, auf dem ein Rechnerprogramm läuft, das die mittels der Stereokamera 20 aufgenommenen Bilder der Marker M des Patienten P in allgemein bekannter Weise auswertet und aufgrund der Auswertung die Position der Marker M und somit die Position des Patienten P im Raum bestimmt.

Im Falle des vorliegenden Ausführungsbeispieles ist die Erfassungsvorrichtung 18 mit ihrer Stereokamera 20 in dem Roboterarm A integriert. Aufgrund dieser Integration ist die Position der Erfassungsvorrichtung 18 im Raum bekannt, wodurch auch die Position des Patienten P im Raum errechnet werden kann.

Im Falle des vorliegenden Ausführungsbeispiels ist vorgesehen, dass der Roboter R mit dem medizinischen Instrument 21 in automatisierter Weise den Patienten P behandelt. Für diese Behandlung läuft auf dem Steuerrechner 17 ebenfalls ein geeignetes Rechnerprogramm. Aufgrund der Position und Orientierung des medizinischen Instrumentes 21, die aufgrund der Position und Orientierung des Roboters R bzw. aufgrund der Position der Erfassungsvorrichtung 18 relativ zum Roboter R bestimmbar ist, und der mittels der Erfassungsvorrichtung 18 ermittelten Position und gegebenenfalls Orientierung des Patienten P ist es dem Roboter R möglich, in vorab vorgegebener Weise das medizinische Instrument 21 derart zu bewegen, dass der Patient P wie gewünscht behandelt wird.

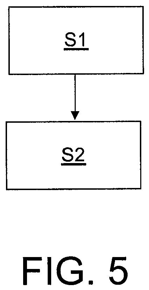

Um die Position und gegebenenfalls Orientierung des Patienten P im Raum zu erhalten, ist im Falle des vorliegenden Ausführungsbeispiels zunächst eine Registrierung der Marker M nötig. Die Fig. 5 veranschaulicht das im Falle des vorliegenden Ausführungsbeispieles verwendete Verfahren zum Registrieren der Marker M.

Im Rahmen der Registrierung wird die Ausgangslage bzw. Ausgangsposition des Patienten P im Raum relativ zum Operationssitus und der Patientenliege L erfasst. Dafür werden im Falle des vorliegenden Ausführungsbeispieles automatisch die Marker M mittels der Stereokamera 20 gescannt, Schritt S1 des Flussdiagramms der Fig. 5.

Im Falle des vorliegenden Ausführungsbeispieles ist es noch vorgesehen, dass aufgrund der aufgenommenen Marker M der Steuerrechner 17 bzw. ein auf dem Steuerrechner 17 laufendes Rechnerprogramm eine Bewegung des Patienten P erkennt. Die Bewegung wird z.B. durch Auswerten der mit der Stereokamera 20 aufgenommenen Bilder erkannt.

Bewegt sich der Patient P stärker als ein vorgegebenes Maß, so aktualisiert im Falle des vorliegenden Ausführungsbeispieles der Roboter R automatisch die Position bzw. Orientierung des Patienten P, Schritt S2 des Flussdiagramms.

Alternativ oder zusätzlich kann es auch vorgesehen sein, die Registrierung des Patienten P und/oder die Bestimmung seiner Position bzw. Orientierung in vorbestimmten Zeitabständen, insbesondere periodisch zu wiederholen.

Im Falle des vorliegenden Ausführungsbeispieles ist ferner am Flansch F eine Beleuchtung 19 angebracht, welche insbesondere den Bereich des medizinischen Instrumentes 21 beleuchtet.

Im Falle des vorliegenden Ausführungsbeispieles ist es noch vorgesehen, mit Hilfe der Stereokamera 20 gewonnene Bilddaten mit einem präoperativ erstellten, insbesondere dreidimensionalen Bilddatensatz vom Patienten P zu kombinieren. Der präoperativ gewonnene, insbesondere dreidimensionale Bilddatensatz wurde beispielsweise mittels eines in der Fig. 4 dargestellten bildgebenden medizintechnischen Geräts 22, beispielsweise eines Magnetresonanzgeräts, eines Computertomographiegeräts, eines Röntgengeräts oder eines Ultraschallgeräts erzeugt.

Im Falle des vorliegenden Ausführungsbeispieles ist die Erfassungsvorrichtung 18 des Navigationssystems in den Roboterarm A integriert. Die Erfassungsvorrichtung 18 kann auch in einem feststehenden Teil des Roboters R, z.B. dessen Sockel S, integriert sein oder auch starr, aber lösbar am Roboter R, insbesondere an dessen Roboterarm A befestigt sein.

Anstelle von Markern M am Patienten P können auch markante Stellen des Patienten P für das Erkennen seiner Position und gegebenenfalls seiner Orientierung verwendet werden.

## Patentansprüche

1. Vorrichtung, aufweisend
- ein Navigationssystem (17, 18), das eine Erfassungsvorrichtung (18) zum Erfassen von markanten Stellen eines Objekts (P) oder von am Objekt (P) angeordneten Marker (M) und eine Verarbeitungsvorrichtung (17) zum Ermitteln der Position des Objekts (P) aufgrund der mit der Erfassungsvorrichtung (18) erfassten markanten Stellen bzw. Markern (M) aufweist, und
- einen Roboter (R), an dem ein medizinisches, insbesondere ein chirurgisches Instrument befestigt ist und der einen Roboterarm (A) mit mehreren Drehachsen (1-6) umfasst, an dem die Erfassungsvorrichtung (18) des Navigationssystems befestigt ist, wobei die Erfassungsvorrichtung (18) integraler Bestandteil des Roboterarms (A) oder starr, aber lösbar am Roboterarm (A) befestigt ist, der Roboter (R) eingerichtet ist, nach bestimmten Zeitabschnitten automatisch die Marker (M) bzw. die markanten Stellen neu zu registrieren, und **dadurch gekennzeichnet ist, dass** der Roboter (R) über redundante Freiheitsgrade verfügt, um eine Änderung des Blickwinkels der Erfassungsvorrichtung (18) bei gleichzeitiger Beibehaltung des Tool Center Points zu ermöglichen.

2. Vorrichtung nach Anspruch 1, bei der die Erfassungsvorrichtung eine optische Erfassungsvorrichtung (18), insbesondere eine Kamera (20), ein Laser-Tracking-System, Projektoren für strukturiertes Licht oder Linienprojektoren aufweist.

3. Vorrichtung nach Anspruch 1 oder 2, aufweisend eine an dem Roboter (R) befestigte Beleuchtung (19).

4. Vorrichtung nach einem der Ansprüche 1 bis 3, dessen Roboter (R) eingerichtet ist, eine Bewegung des Objekts (P) zu erkennen und aufgrund der erkannten Bewegung automatisch die Positionen der Marker (M) bzw. die markanten Stellen zu aktualisieren.

5. Vorrichtung nach einem der Ansprüche 1 bis 4, dessen Roboter (R) eingerichtet ist, die Marker (M) bzw. die markanten Stellen durch Anfahren und/oder durch Aufnehmen zu registrieren.

6. Arbeitsplatz, aufweisend eine medizintechnische Vorrichtung nach einem der Ansprüche 1 bis 5 und eine Patientenliege (L), die vorgesehen ist, dass auf ihr ein Lebewesen (P) als das Objekt liegt.

7. Verfahren zum Registrieren eines Objekts, aufweisend folgenden Verfahrensschritt:
- automatisches Registrieren von markanten Stellen eines Objekts (P) oder von an der Oberfläche des Objekts (P) angeordneten Markern (M) mittels eines Roboters (R), an dem ein medizinisches, insbesondere ein chirurgisches Instrument befestigt ist und der einen Roboterarm (A) umfasst, an dem eine Erfassungsvorrichtung (18) eines Navigationssystems zum Erfassen der markanten Stellen bzw. der am Objekt (P) angeordneten Marker (M) befestigt ist, wobei das Navigationssystem eine Verarbeitungsvorrichtung (17) zum Ermitteln der Position des Objekts (P) aufgrund der mit der Erfassungsvorrichtung (18) erfassten markanten Stellen bzw. Marker (M) aufweist und die Erfassungsvorrichtung (18) integraler Bestandteil des Roboterarms (A) oder starr, aber lösbar am Roboterarm (A) befestigt ist, und
- nach bestimmten Zeitabschnitten neues automatisches wiederholtes Registrieren der Marker (M) bzw. der markanten Stellen, wobei der Roboter (R) über redundante Freiheitsgrade verfügt, um eine Änderung des Blickwinkels der Erfassungsvorrichtung (18) bei gleichzeitiger Beibehaltung des Tool Center Points zu ermöglichen.

8. Verfahren nach Anspruch 7, ferner aufweisend automatisches Erkennen einer Bewegung des Objekts (P) und aufgrund der erkannten Bewegung des Objekts (P), automatisches Aktualisieren der Positionen der der Marker (M) bzw. der markanten Stellen.

9. Verfahren nach Anspruch 7 oder 8, aufweisend automatisches Aufsuchen der markanten Stellen bzw. der Marker (M) durch den Roboter (R), um die Marker (M) bzw. die markanten Stellen zu registrieren, und/oder aufweisend automatisches Aufnehmen der markanten Stellen bzw. der Marker (M), um die Marker (M) bzw. die markanten Stellen zu registrieren.

10. Verfahren nach einem der Ansprüche 7 bis 9, bei dem die Erfassungsvorrichtung eine optische Erfassungsvorrichtung (18), insbesondere eine Kamera (20), ein Laser-Tracking-System, Projektoren für strukturiertes Licht oder Linienprojektoren aufweist.

## Claims

1. Device comprising
- a navigation system (17, 18) which comprises a detecting device (18) for detecting distinctive points of an object (P) or markers (M) associated with the object (P) and a processing device (17) for determining the position of the object (P) on the basis of the distinctive points or markers (M) detected by the detecting device (18), and
- a robot (R) to which a medical, in particular a surgical instrument is secured and which comprises a robot arm (A) having a plurality of axes of rotation (1-6), on which the detecting device (18) of the navigation system is secured, wherein the detecting device (18) is an integral part of the robot arm (A) or is secured rigidly but detachably onto the robot arm (A), the robot (R) is configured to re-record the markers (M) or the distinctive points automatically at specific time intervals and is **characterised in that** the robot (R) has redundant degrees of freedom in order to allow a change in the perspective of the detecting device (18) while simultaneously maintaining the tool centre point.

2. Device according to claim 1, wherein the detecting device comprises an optical detecting device (18), in particular a camera (20), a laser tracking system, projectors for structured light or line projectors.

3. Device according to claim 1 or claim 2, comprising a light (19) secured to the robot (R).

4. Device according to any of claims 1 to 3, the robot (R) of which is set up to identify a movement of the object (P) and from the identified movement to automatically update the positions of the markers (M) or the distinctive points.

5. Device according to any of claims 1 to 4, the robot (R) of which is configured to record the markers (M) or the distinctive points by moving towards them and/or recording the latter.

6. Workstation comprising a medical technical device according to any of claims 1 to 5 and a patient couch (L) which is provided so that a living being (P) can lie on the latter as the object.

7. Method for recording an object comprising the following method step:
- automatically recording distinctive points of an object (P) or markers (M) arranged on the surface of the object (P) by means of a robot (R) to which a medical, in particular a surgical instrument is secured and which comprises a robot arm (A) on which a detecting device (18) of a navigation system is secured for detecting the distinctive points or the markers (M) arranged on the object (P), wherein the navigation system comprises a processing device (17) for determining the position of the object (P) on the basis of the distinctive points or markers (M) detected by the detecting device (18) and the detecting device (18) is an integral part of the robot arm (A) or is secured rigidly but detachably to the robot arm (A) and
- after specific time intervals re-recording the markers (M) or the distinctive points automatically, wherein the robot (R) has redundant degrees of freedom in order to allow a change in the perspective of the detecting device (18) while simultaneously maintaining the tool centre point.

8. Method according to claim 7, also comprising automatically detecting a movement of an object (P) and on the basis of the detected movement of the objet (P) automatically updating the positions of the markers (M) or the distinctive points.

9. Method according to claim 7 or 8, comprising automatically seeking the distinctive sites or the markers (M) with the robot (R) in order to record the markers (M) or the distinctive points and/or comprising automatically recording the distinctive sites or the markers (M) in order to record the markers (M) or the distinctive points.

10. Method according to any of claims 7 to 9, in which the detecting device comprises an optical detecting device (18), in particular a camera (20), a laser tracking system, projectors for structure light or line projectors.

## Revendications

1. Dispositif présentant
- un système de navigation (17, 18) qui présente un dispositif de détection (18) pour détecter des emplacements marquants d'un objet (P) et/ou des marqueurs (M) agencés sur l'objet (P) et un dispositif de traitement (17) pour déterminer la position de l'objet (P) sur la base des emplacements marquants et/ou des marqueurs (M) détectés avec le dispositif de détection (18), et
- un robot (R) sur lequel est fixé un instrument médical, en particulier chirurgical, et qui comprend un bras de robot (A) avec plusieurs axes de rotation (1-6), sur lequel est fixé le dispositif de détection (18) du système de navigation, le dispositif de détection (18) faisant partie intégrante du bras de robot (A) ou étant fixé à demeure mais de manière détachable sur le bras de robot (A), le robot (R) étant adapté pour enregistrer de nouveau automatiquement les marqueurs (M) ou les emplacements marquants, respectivement, après des intervalles de temps déterminés, et qui est **caractérisé en ce que**
le robot (R) dispose de degrés de libertés redondants pour permettre une modification de la perspective du dispositif de détection (18) tout en maintenant en même temps le Tool-Center-Point.

2. Dispositif selon la revendication 1, dans lequel le dispositif de détection présente un dispositif de détection (18) optique, en particulier une caméra (20), un système laser tracker, des projecteurs pour lumière structurée ou des projecteurs à ligne.

3. Dispositif selon la revendication 1 ou 2, présentant un éclairage (19) fixé sur le robot (R).

4. Dispositif selon l'une des revendications 1 à 3, dont le robot (R) est configuré pour reconnaître un mouvement de l'objet (P) et pour actualiser, sur la base du mouvement reconnu, automatiquement les positions des marqueurs (M) et/ou des emplacements marquants.

5. Dispositif selon l'une des revendications 1 à 4, dont le robot (R) est configuré pour enregistrer les marqueurs (M) et/ou les emplacements marquants, par approche et/ou prise de vue.

6. Poste de travail, présentant un dispositif médico-technique selon l'une des revendications 1 à 5 et une table médicale (L) qui est prévue pour recevoir un être vivant (P) en tant qu'objet.

7. Procédé d'enregistrement d'un objet, présentant l'étape de procédé suivante :
- enregistrement automatique d'emplacements marquants d'un objet (P) ou de marqueurs (M) agencés sur la surface de l'objet (P) au moyen d'un robot (R) sur lequel est fixé un instrument médical, en particulier chirurgical, et qui comprend un bras de robot (A) sur lequel est fixé un dispositif de détection (18) d'un système de navigation pour détecter les emplacements marquants (M) et/ou les marqueurs (M) agencés sur l'objet (P), le système de navigation présentant un dispositif de traitement (17) pour déterminer la position de l'objet (P) sur la base des emplacements marquants et/ou marqueurs (M) détectés avec le dispositif de détection (18), et le dispositif de détection (18) faisant partie intégrante du bras de robot (A) ou étant fixé à demeure ou de manière détachable sur le bras de robot (A), et
- après des intervalles de temps déterminés, nouvel enregistrement automatique répété des marqueurs (M) ou des emplacements marquants, respectivement, le robot (R) disposant de degrés de liberté redondants pour permettre une modification de la perspective du dispositif de détection (18) tout en maintenant en même temps le Tool-Center-Point.

8. Procédé selon la revendication 7, présentant en outre la reconnaissance automatique d'un mouvement de l'objet (P) et, sur la base du mouvement reconnu de l'objet (P), l'actualisation automatique des positions des marqueurs (M) et/ou des emplacements marquants.

9. Procédé selon la revendication 7 ou 8, présentant la recherche automatique des emplacements marquants et/ou marqueurs (M), par le robot (R) pour enregistrer les marqueurs (M) ou emplacements marquants, et/ou présentant la prise de vue automatique des emplacements marquants ou marqueurs (M), pour enregistrer les marqueurs (M) ou emplacements marquants.

10. Procédé selon l'une des revendications 7 à 9, dans lequel le dispositif de détection présente un dispositif de détection (18) optique, en particulier une caméra (20), un système laser tracker, des projecteurs pour lumière structurée ou des projecteurs à ligne.
